# EUROPEAN PATENT APPLICATION

(11) **EP 1 130 037 A1**
(43) Date of publication of application: **05.09.2001**
(21) Application number: 99934450.0
(22) Date of filing: 19.07.1999
(51) Int. Cl.: C08F 6/00, C08F 6/08, C08F 8/12, C08F 118/08, C08F 218/08, C08J 3/12, A61K 47/32

(54) **PROCESS FOR OBTAINING POLYMERS DERIVED FROM VINYL ACETATE, AND USES THEREOF**

(30) Priority: 17.07.1998 CU 10098
(71) Applicant: Centro Nacional De Investigaciones Cientificas (CNIC), Ciudad de la Habana 12100 (CU)
(72) Inventor: SUZARTE PAZ, Alberto Guillermo, C. Duany No. 3 e, Arroyo Naranjo, Ciud. de la Habana 13800 (CU); ECHEVERRIA PEREZ, Mayda Luisa, Aven. 23a No. 23020, Ciudad de la Habana 13600 (CU); IGLESIAS RODRIGUEZ, Gerardo, Ciudad de la Habana 10400 (CU); DIAZ AGUILA, Elsa Eneida, Avenida 29 No. 23814, Ciudad de la Habana 13600 (CU); JORDAN MARTINEZ, Guillermo, Ciudad de la Habana 11400 (CU)
(74) Representative: Davila Baz, Angel
(86) International application number: CU9900002
(87) International publication number: WO0004060

(57) **Abstract**

Process for obtaining polyvinyl acetate (Mw = 10 000-40 000 daltons) and copolymers of Vac-VA with a molar content of monomer units of vinyl alcohol lower than 30 %, high purity and innocuous by oral administration and utilization of the same as agglutinating agents and exclusive or fundamental constituents of matrices which control the release of active principles in tablets and pellets. The PVAc and the copolymers can be transformed into powders and granules as a base with acceptable stability during its shelf life.

## Description

### Technical sector

This invention is directed to the obtaining of polymers and the use of these in the production of oral solid preparations such as tablets, pills, and pellets, that present or not retarded, controlled or sustained therapeutic action in human or veterinary medicine. It also is directed to all the possible use fields in which starting from solid preparations, an active principle is delivered to an aqueous medium with a controlled delivery rate.

In the international industrial practice the use of synthetic polymers in the quality of binders for the production of tablets and other solid preparations has won wide diffusion, because of their relatively low costs and the high quality of the products, competing efficiently with natural and semi-synthetic polymers. Example of this is the growing use of polyvinylpyrrolidone.

On the other hand at this moment exist in the scientific and patent literature and in the international market of pharmaceutical oral solid products numerous examples of solutions to the problem of achieving that a drug or active substance is delivered at such a rate that the receiving organism disposes an appropriate quantity for a more or less long period of time for a better therapeutic effect. To achieve this, several technological solutions have been used, such as
·- Controlled diffusion through matrixes and permeable membranes.
·- Slow formation of a hydrogel owed to the swelling of the occluding agent and diffusion of the active principle through the gel.
·- Diffusion of the active principle through a hole in a permeable membrane to the aqueous media that surrounds a complex tablet, with a nucleus of very high content of active (osmotic pump principle).
   - Erosion of the active substance occluding agent due to slow dissolution..
   - Erosion of the occluding agent by means of chemical or biological degradation.
·- Combination of several principles.

Numerous substances have been used for these purposes, standing out among them the natural and synthetic polymers.

The polyvinylacetate and the vinyl acetate - vinylalcohol copolymers with less than 30% molar vinyl alcohol content are synthetic polymers that present some advantageous properties that support a priori the perspective of its use in pharmaceutical formulations solid. It should be mentioned among these characteristics:
- The innocuousness that is to expect from their chemical structure.
- The relative low cost of the starting materials for their synthesis.
- The possibility to develop simple obtaining processes.
- Their insolubility in aqueous media although they are highly hydrophilic.

The fact that these polymers have a vitreous transition temperature near to 40 °C. This implies that if they are used in the composition of tablets the heat that is generated at the compression could be enough to reach the vitreous transition temperature so that the polymer can flow through all the interstices, forming a continuous matrix. This continuous matrix can be achieved even when the polymer constitutes a very minor fraction of the formulation.

### Background of the invention

In the scientific- technical- and patent literature there are a considerable number of examples of using polyvinylacetate in certain individualized pharmaceutical formulations, mainly in conjunction with other polymers for the achievement of binding or release controlling effects. The most important references are listed below:
A) Sustained release tablets of theophylline with employment of PVAc-PVP matrixes ( Ortega A., Avenida M ; Eur. Pat. Appl. E. P. 231862 (1987)).
B) Theophylline microspheres with sustained release action using PVAc (50 - 70 % content of the polymer) (Biswanath S. ; Drug Development and Industrial Pharmacy , 17 (6) 893-900 (1991))
C) Paracetonal tablets using copolymers of vinyl acetate and vinyl pirrolidone as binder (Barlow Chistopher S. African 73 03, 289 (1974) C. A. 88 15665).
D) Pharmaceuticals for oral applications with retarded action prepared by deposition of the drug on sugar or starch pellets and subsequent coating with a dialyzing membrane that contains PVAc and ethylcelullose (Fuelbecht W., Neitzger E.; Ger. Offen. 2010416 (1971), C.A. 75 144002).
E) Incorporation and release of theophylline in PVAc matrix in form of granulates (not tablets or pellets) of different particle size (Huang Jin Din, Gan Tz Huang; Tái.wanYao HsuehTsa Chi, 38, (3) 160-165 (1986)).
F) Depot medicaments in capsule form for oral use in which poliethylenglycol is used as carrier, PVAc in ethanol as sponge former and active agents, everything in capsules of gelatine (Scherer R.P. GmbH, Brit. 1242547 (1971); C.A. 75 121403)
G) Coating compositions for compressed tablets using polyethyleneglycol, sugar, PVAc and water insoluble fillers (Boehringer C.F.und Soehne GmbH; Fr. 1588833; (Cl A61J), C.A. 73 123520).
H) PVAc emulsions as carriers in sustained release drugs (Takebe T., Maebutzu T., Ito K., Maekawa M.; Jp 7312,069 (C1C08fA61K) 1973; C.A. 80, 52394).
I) Preparation of agglomerated crystals for direct tabletting and microecapsulation by the spherical crystalization technique with a continuous system, using PVAc and ethylcellulose as coating polymers (Niwa T., Takeuchi H., Hino T., Itoh A. Kawashima Y., Kiuchi K.; Pharmaceutical Research (New York) 11(4): 478-484 (1994)).
J) Sustained release oral pharmaceutical films containing vinyl pirrolidone and vinyl acetate polymers (Kuroya Takamasa, Jpn Kokai Tokkyo Koho J.P. 01,268630 [89,2268,630] (1989)).
K) Films of hydroxipropylcellulose - PVAc for the controlled release of active substances (Borodkin S., Tucker F. E.; J.; Pharm. Sci. 64 1289 (1975); J. Pharm. Sci. 63 1359 (1974)).
L) Controlled delivery of iron ions by means of pelletization of the sulfate salt and coating of the pellets with PVAc (Batra V. et al.; J. Pharm. Sci., 83(5) p. 632 (1994)).
M) Complex oral delivery system with microspheres that can float in gastric juice and which are coated with PVAc and purified shellac (Ichikawa M., Watanobe S., Miyake Y.; J. Pharm. Sci., 80 (11) 1062, (1991)).
N) Pharmaceutical formulations in form of powders, granulates and tablets based in the use of chitin with addition of PVAc (J. P. 62016413, 19870124).
O) Base granulate for pharmaceutical formulations with controlled action employing two kinds of polymers; polymer A (hydroxipropylcellulose or methylcellulose or polyvinylpirrolidone) and polymer B (ethylcellulose or a copolymer of methacrylate or PVAc) (J.P. 04074137 19920309
P) Tablet formulation for direct compression containing a mixture of polyvinylpirrolidone and PVAc as binder and aerosil as lubricant (Tawashi R. Pharm. Ind. 26, 10, 682 (1964), C.A. 62 5147).

The uses of PVAc and vinylacetate - vinylalcohol copolymers in the discussed references can be summarized as follows;
- PVAc has been proposed to be used in controlling matrixes or as binder, in conjunction with other polymers which contribute also to the controlling and binding action (references A , C , D , G , K , L , N , O , P ).
- In reference B the PVAc is used as the only controlling substance in microspheres of theophylline which present a high content of polymer and therefore should be kept under dry and cool conditions since otherwise evidently agglomerate. Similar is proposed in reference E), where PVAc is used in granulates of theophylline with some lower polymer content. Compressing the granulates is not proposed in E).
- Other references deal with the use of PVAc as coating material, alone or in conjunction with other polymers, of tablets, pellets or microspheres (references D, G, I, L).
- The Cuban Patent No. 22199 (1993), with the title "Polymers derived from vinyl acetate as binders and polymeric matrixes in oral sustained release formulations", claims the use of PVAc with molecular masses between 10000 and 200000 daltons and residual monomeric content of less than 10 ppm, and copolymers of vinyl acetate vinyl alcohol with less than 50 % (molar) vinyl alcohol monomeric units.
- None of the discussed references mentions the quality requirements of PVAc or co (VAc-VA), regarding to its safe use when used in pharmaceutical oral formulations for humans.

The existent regulations of important countries such as United States or Japan do not authorize the use of polyvinylacetate in drugs or food for humans, in which the polymer should be swallowed. The North American (USA) F.D.A. and Food Chemical Codex authorize only the use of PVAc with a certain degree of purity for packing materials and coating in contact with food (F.C.C. (1981) Third Edition , Washington D.C. , National Academy Press , page 237) and as masticatory for chewing gum (F.D.A. 21 CFR Ch. (4-1-94 Edition ) , paragraph 172615 ). In Japan the use of PVC is authorized also in chewing gum and as coating material for fruits and vegetables (The Japanese Standards for Food Additives, Sixth Edition, Japan Food Additive Association, 1994, ps. 478, 649 and 653).

Although there are, as discussed, many references to the possibilities to use polyvinylacetate in pharmaceutical formulations, this polymer practically is not been used by the pharmaceutical industry. The mainly reasons for this are the following:
- Generally the commercial polymers show a high content of impurities, particularly residual monomer up to 200 ppm, (U.C.C. (1989) Material safety data sheets for vinyl acetate polymers. Submission of data to C.T.F.A. Available upon request: Director Cosmetic Ingredient Review 1101 17th street N.W. Suite 110 Washington D.C. 20036). The oncogenic character of the monomer through inhalation has been reported and proved (Sanotsky I.V., Unlanova I.P. United Nations Enviroment Program. Centre of International Projects G.K.N.T. Moscow 1983) (Bogdanffy M. S., et al; Fundamental and Applied Toxicology 23, 215-229 (1994) ).and the toxic character have been extended also to oral intake on account of the results presented by Lijinski (Lijinski W. ; Reuber M. D.; Toxicol. Appl. Pharmacol. 63, 43-53 (1983)) Later has been stated that vinyl acetate does not present the presumed toxicity when is administered orally in not very high doses ( Clary J., "Annals of the N.Y. Acad. of Sciences", Vol 534 pag 225 (1988), (Bogdanffy F.M.B., Casciere T.C., Fundamental and Applied Toxicology 23, 206-214 (1994) ). Differently to the vinyl halides, that are decomposed in their metabolism in epoxidic intermediaries, vinyl acetate is decomposed hydrolitically, by the acid of the stomach and by enzymes (sterases) in intestine and blood, rapidly into acetaldehyde and acetic acid (Simon P., J.G. Filser, H.M.Bolt: "Metalbolism and Pharmacokinetics of vinyl acetate", Arch. Toxicol. 57, 191-195. (1985)), (Kuykendall J.R., Bogdanffy M.S.; Carcinogenesis 1992. Nov. 13 (11) 2095-2100), (Kuykendall J.R., Taylor M.L., Bogdanffy M.S.; Toxicol. Appl. Pharmacol. 1993 Dec. 123(2) 283-92).
- The reported studies of oral toxicity of PVAc are not conclusive, since the studied polymers were impure with remnant monomer, thickening agent, surfactant and plastizisers. The adverse effects found in those studies were attributed by the authors of the mentioned studies to the impurities (particularly to the plastiziser present) ( Scherbak BI. et al " Uch. Zap.-Mosk. Nauch. Issled. Inst. Gig. 22, 74-80 (1975); I.A.R.C. Vol. 19 pag. 351), (Scherbak B.I.,; Gig. Sanit. 4:99 (1977)). Although it is to expect from their chemical structure that PVAc and the mentioned copolymers are completely innocuous in oral formulations, new studies are necessary to demonstrate this (Final Report on the Safety Assessment of Polyvinyl Acetate, Journal of the American College of Toxicology Vol. 11 Number 4 1992 Mary Ann Liebert Inc. Publishers).
- The development of simple and reliable obtaining technologies is required in order to guarantee polymers of high purity and reproducible composition at low costs.
- It is required a deep understanding of the mechanisms that control the diffusion of occluded substances in the said polymers prior the generalization of their use in pharmaceutical formulations.

### - Description of the Invention

Taking into account the previously exposed criteria the essence of the present invention is based on:
- New technological procedure for obtaining polyvinilacetate of molecular mass (Mw) between 10000 and 40000 daltons, high purity, remnant monomer content less than 2 ppm, humidity less than 1.5 weight %, and safe for oral ingestion, characterized by a novel purification and drying procedure
- New procedure for obtaining vinyl acetate - vinyl alcohol copolymers with predetermined composition by means of alkaline alcoholysis of less than 30 % of the acetate groups present in the polyvinylacetate used as starting material.
- New procedure for obtaining polyvinylacetate and vinylacetate - vinyl alcohol copolymers containing powders and granulates of acceptable physical stability during their storage at ambient temperatures and suitable for use in pharmaceutical formulations, characterized by a initial milling of the polymers to obtain a gross grained product and a final fine milling of the said product together with solid powdery crystalline excipients of small particle size usually used in pharmaceutical solid formulations , preferably lactose . This procedure could be also employed in the preparation of powders or granulates in which the polymer is mixed with crystalline active substances with melting temperatures higher than 60 °C.
- Use of polyvinylacetate (Mw: 10000 - 40000, high purity, remnant monomer content < 2 ppm) as the only or principal mean of agglutination in tablets, pellets or pills used in human or veterinary medicine in contents between 1 and 10 % by weight of the formulation.
- Use of polyvinylacetate (Mw: 10000 - 40000, remnant monomer content < 2 ppm) and copolymers of vinyl acetate- vinyl alcohol with less than 30 % (molar) vinyl alcohol monomeric units and same purity requirements as said poyvinylacetate as the only or principal factor for the achievement at the same time of the required binding effect and the control of the delivery rate of active substances in slow or controlled release solid preparations (tablets, pellets or pills) for use in human or veterinary medicine with said polymer or copolymer contents between 5 and 40 % by weight of the formulation or preparation.

The essential aspects of the present invention are:

### 1 TECHNOLOGICAL PROCEDURE FOR OBTAINING POLYVINYLACETATE OF ADEQUATE MOLECULAR WEIGHT AND PURITY

The procedure is characterized by the following technological steps:
1.1 Obtaining of polyvinylacetate by means of Tadical polymerization of vinyl acetate in suspension or solution using dibenzoyl peroxide as initiator taking place the reaction in presence of an alcohol, preferably ethanol, that acts not only as solvent but also as chain transference agent that controls the average molecular weight. The reaction temperature is kept constant in the range of 55-90 for reaction times between 5 and 9 hours in dependence of the temperature and the chosen concentrations of monomer and initiator Yield 80-93% molar.
**1.2** Purification of the polymer by means of the addition to the solution resulting from the polymerization reaction of an adequate volume of water, keeping the mixture hot (>80 ° C) and mechanically stirred, or by means of the addition of the said solution to an adequate volume of hot water (>80 ° C), kept hot during and after the addition and mechanically stirred. Due to the high temperature and the stirring (both kept for a time that depends on the characteristics of the employed equipment) takes place the evaporation of the solvent, the decomposition of the remnant initiator, the dissolution in the aqueous phase of the major part of the benzoic acid resultant of the decomposition of the initiator and the elimination of the major part of the remnant monomer, which evaporates together with the solvent and water vapors. Finally and optionally a purified air stream is passed through the liquid keeping the temperature in the range of 80-100 ° C for a better elimination of traces of monomer. After this, the remaining hot water (normally more than the half of the added water), is separated. This water contains the majority of the benzoic acid present. New hot water can be added and separated after some stirring in order to eliminate more benzoic acid. This is not indispensable, since dibenzoyl poeroxide and benzoic acid are considered safe food additives by the F.D.A. of U.S.A., (F.D.A.: 21 CFR Part 184 Subpart B; 21 CFR184 1157; 21 CFR paragraph. 184.1021), (The Food Chemical Codex, 3^{rd} Eddition1981 p. 35)

The semisolid mass, that contains the polymer and about 30 % of water (by weight), is heated to a temperature in the range of 80-140 ° C and stirred slowly in vacuum (0.02-13 kPa) as long as necessary to obtain the polymer with less than 1.5 % humidity and 2 ppm of remnant monomer. The so purified polymer is taken out molten from the purifying equipment and can be dissolved (preferably in acetone or ethanol) or cooled and subsequently milled, in dependence of the intended use of it.

This technology showed itself efficient in the substantial reduction of the remnant monomer and humidity. The remnant monomer should be separated, not only from the toxicological point of view, but also in order to avoid possible undesirable interactions with drugs, active substances and the other excipients of the pharmaceutical preparations. On the other hand, if the water content of the polymer is higher than the limit previously mentioned, the polymer could not be milled, since it would show plastic properties at room temperature. The described procedure for purification and drying is technologically the simplest and most efficient. The drying for example with a static procedure in vacuum ovens is too slow and requires big and very expensive equipment. This is determined by the fact that the width of the polymer layer on the trays should be small, because otherwise the wet inflated polymer would overflow the trays at the beginning of the drying process. Is the width of the polymer layer not thin enough, then the drying process would last many hours or days.

The proposed procedure has however its own limitations because it requires the spending of much energy to move the very high viscous polymer, especially when the polymer is almost dry. (see Fig. 1). For the measurement of the viscosity was used a rotatory viscometer with a cylinder device. The existence of a structural viscosity behavior for the wet polymer can be concluded from Fig. 2. For the dry polymer such a behavior does not result evident (see Fig. 3)

This means that even under an agitated regime the viscosity rises to very high values when the polymer approaches to the dryness and purity condition.

The increase of the temperature can determine a substantial lowering of the value of the viscosity. The dependence of the logarithm of the viscosity of a PVAc sample (Mw 0 27500) vs, the inverse of the absolute temperature shows a linear behavior (r = 0.999). The energy of activation of the viscous flow (E) and the preexponential factor (K) for the temperature range of 100-125 °C are E= 106693 J and K= 21.19589.

The increase of the temperature has however a practical limit imposed by the stability of the polymer. The thermal stability study of different polymer samples was carried out by means of the thermolysis of the said samples in a mass spectrometer with programmable temperature in the sample introduction chamber (8 °, 16° and 32°C/min). The decomposition vapors were conducted to the ionization chamber and the intensity of the total ionic current and of the current owed to the ion M/Z = 60 (acetic acid) were registered at all temperatures of the introduction chamber. The mass spectra were also registered.

For dry PVAc samples the thermograms for total ionic current obtained at 8, 16, and 32 ° C/min showed no differences. At temperatures below 120° C the thermal decomposition is negligible. The mass spectra in this temperature range only show the presence of traces of benzoic acid (from the decomposition of the iniciator used in the polymerization) and that is partially desorbed under the high vacuum. This impurity is totally desorbed at temperatures between 120 and 130° C), starting in this temperature range a slow degradation of the polymer with formation of acetic acid. A similar thermogram obtained from a sample of PVAc with a water content of 2 % by weight (heating rate 8° C / min.) evidenced that the presence of water does not induce a sensible hydrolytic degradation at temperatures below 120 ° C.

Taking into account that the viscosity depends directly from the molecular mass and that it can not be lowered increasing the temperature over 130- 140 ° C without degrading the polymer, it is necessary that the obtained polymer has an average molecular mass low enough to allow technologically its easy purification and drying.

From the practical point of view the limit of the average molecular mass is approximately 40000 daltons. On the other hand it has been proved that polymers with 10000<Mw < 40000 are completely proper for the use as binders or as major constituents of release controlling matrixes.

The polymers obtained by the described procedure fit the following specifications:
Remnant monomer: < 2 ppm by weight (contents less than 0.5 ppm are achievable)
Total acidity referred as acetic acid: <0.5 % by weight (fundamentally benzoic acid)
Water: < 1,5 % by weight.
Peroxides: 0.0 %
Glass transition temperature: 35-39° C.

The polyvinylacetate obtained by the described process presents levels of impurities that are similar or lower to the requirements established by the North American F.D.A. and the Food Chemical Codex or The Japanese Standards for Food Additives for the use of this polymer in packing materials and coatings in contact with food or for use as masticatory in chewing gums. In the case of remnant monomer the levels of content in the polymer obtained by the described process are much lower than the upper permitted level in the mentioned requirements.

The toxicity of the polyvinylacetate obtained by means of the described process was studied performing the following tests and studies:
- Citotoxicity test (test of isolated rat hepatocites): PVAc at a dose of 5 or 10 mg did not show any citotoxic effect.
- Acute oral toxicity study and chronic oral toxicity studies (one three and six months).
   - Single dose study (acute). Three male and three female rats for each PVAc dose level (250, 2000, 5000 y 10000 mg/kg body weight). The PVAc was administered incorporated in the food pellets that were totally consumed by the rats.
   - Repeated dose study (chronic) (one month) 40 animals for each group (20 males and 20 females). Three groups consuming the substance with the food in three dose levels (100,750 y 2500 mg/kg body weight), one control group.
   - Repeated dose study (chronic) (thee and six months).

Three months study: 30 animals (15 males and 15 females) for each dose level (125, 250 y 2500 mg/Kg body weight) and the same number of animals for a control group.

Six months study: 20 animals for each group (10 males and 10 females). Three groups consuming the substance with the food in three dose levels (125, 250 y 2500 mg/kg body weight), one control group and a sentinel group.

Sprangue Dawley were used in these studies and they were first feed every day with food with incorporated polymer and only after this food was completely consumed they were allowed to consume normal food ad libitum.

The following parameters were evaluated:
- Hematological parameters.
- Biochemical parameters.
- Measurements of acetaldehyde in blood.

The following studies were performed:
- Histopatological studies
- Microbiological studies
- Clinical evaluation.
- Electron microscopy structural study of the small and large intestine (10 animals, 5 of the control group and 5 treated after the 6 months study)

The results of the mentioned tests and studies showed that the PVAc obtained by the described procedure is not citotoxic and that no signs or symptoms of toxicity were found which could be related with the oral intake of the polymer. These results are complemented with studies of citotoxicity and genotoxicity reported in the scientific literature (Ishidate M.J. Jr. et al. "Primary mutagenicity screening of food additive currently used in Japan" Food Chem. Toxicol. 22(8): 623-636 (1984)). It is therefore possible to conclude that the polymer is innocuous when is orally administrated.

### 2-NEW OBTAINING PROCEDURE OF VINYL ACETATE-VINYL ALCOHOL COPOLYMERS WITH LESS THAN 30 MOLAR % OF VINYL ALCOHOL UNITS.

Using the polyvinylacetate as starting material it is possible to obtain through different ways copolymers of vinyl acetate - vinyl alcohol. With a vinyl alcohol monomeric unit content between 0 and 30 molar %. The transesterification reaction using an alkaline catalyzer represents the best choice to synthesize copolymers with certain block character. It is known that the presence of water in this kind of reactions exerts control of the extent of the alcoholysis. The addition of controlled quantities of water (in concentrations between 2 and 20 % of the PVAc concentration) to solutions of PVAc in NaOH containing methanol at temperatures between 10 and 40 ° C, allows to control the methanolysis/hydrolysis yielding copolymers with a vinyl alcohol monomeric molar content between 93 and 75 % (Dimonie M y cols; Rom. 58,176 (Cl. C08F 27/ 16), 25 Mar. 1975; Appl. 67,621 12 Jul. 1971).

The here proposed procedure is characterized by also the use of an alcohol as solvent, by the establishment of a proportion between the initial quantities of polyvinylacetate / NaOH / solvent and by the addition to the initial reaction mixture of water quantities that represent between 4 and 25 % of the solvent present, to yield the desired copolymers being unnecessary to stop the reaction at any time. The reaction time is less than 30 minutes. The mass ratio between PVAc/alcohol/NaOH must be kept in the range 50-80 / 130-170 / 1 respectively. The procedure is characterized by the instantaneous mixing, using mechanical stirring, of two solutions, both at the same temperature in the range between 20 and 40 ° C. One of the solutions is a solution of PVAc in the alcohol with 0-7 % of water. The second solution is a solution of NaOH in the alcohol with a certain quantity of water which added to the quantity of water present in the first solution gives the desired water quantity necessary for the control of the reaction and therefore of the composition of the obtained copolymers.

### 3 PROCEDURE OF PREPARATION OF POLYVINYLACETATE, AND COPOLYMERS OF VINYL ACETATE - VINYL ALCOHOL, IN FORM OF POWDER SUFFICIENTLY STABLE TO THE STORAGE AT ROOM TEMPERATURE.

PVAc and copolymers of vinyl acetate - vinyl alcohol with block tendency and molar content of vinyl alcohol monomeric units less than 30 % presents in pure state, glass transition temperatures (Tg) between 39 and 44 C. The presence of small quantities of water or residues of organic solvents lessens the Tg to values frequently near room temperature. If this happens, any powder of these polymers would compact during the storage. On the other hand milling of these polymers is extraordinarily difficult, because of the heating generated by the milling process, which determines its compactness and even melting.

A procedure is claimed for obtaining these polymers in powder form, usable in formulations of tablets made by direct compression or wet granulation processes, which is characterized by being executed in three steps, being the first one the milling of the polymer in cutting or hammer mill with sieves between 2,5 and 0,8 mm, and the second the mixing of the gross grained polymer particles obtained in the first step, with a crystalline pharmaceutical excipient of small particle size (preferably lactose) or a crystalline active substance and further milling of this mixture in the same mill, using sieves between 0,2 and 0,04 mm. In the third step is the homogenizing mixing of the milled product.

If the original polymer presents a humidity smaller than 1.5 % in weight, the final product presents, provided it is stored protected from humidity and at temperatures below 30 C, an acceptable physical stability and it doesn't agglutinate during its storage for one year. Even in case it agglutinates because of high room temperatures, it is easy to destroy the agglomerates by fast milling, as it is a usual practice with the raw materials in the pharmaceutical industry previously to granulation.

### 4 USE OF POLYVINYLACETATE OF MASS AVERAGE MOLECULAR WEIGHT FROM 10000 TO 40000 DALTON AND RESIDUAL MONOMER CONTENT LOWER THAN 2 PPM AS THE ONLY OR PRINCIPAL MEAN OF AGGLUTINATION IN TABLETS

Agglutinative agents or binders, play a fundamental role in the formation of tablets, because they are responsible, together with the compression, for providing the necessary cohesive so that the particles or granules form the aggregate solid structure characteristic in tablets, maintaining it after the compression forces have been suspended.

Polyvinylacetate possesses those agglutinative properties. The use of polyvinylacetate with mass average molecular weight between 10000 and 40000 Dalton and residual monomer content lower that 2 ppm is claimed, for using it as binder in procedures of obtaining basic granulates, suitable to be used in the production of tablets by direct or double compression processes, or in formulations with active substances characterized by:
- being obtained from polyvinylacetate in powder form, in intimate mixture with lactose or another crystalline excipient by means of moistening with acetone or ethanol or another appropriate solvent, or being obtained from solid excipients moistened with a polyvinylacetate solution in a suitable organic solvent.
- containing between 1 and 10 % of polyvinylacetate by weight.

### 5 USE OF POLYVINYLACETATE OF MASS AVERAGE MOLECULAR WEIGHT FROM 10000 TO 40000 DALTON AND RESIDUAL MONOMER CONTENT LOWER THAN 2 PPM OR VINYLACETATE - VINYLALCOHOL COPOLYMERS WITH LESS THAN 30 % MOLAR UNITS OF VINYLALCOHOL, AS THE ONLY OR PRINCIPAL MEAN OF AGGLUTINATION AND CONTROL OF THE RELEASE OF ACTIVE SUBSTANCES IN TABLETS AND PELLETS.

Copolymers which are claimed as the only or principal constituents of matrixes for the controlled release of occluded substances are limited to those with less than 30 % of vinyl alcohol monomeric units, because copolymers with a higher content of vinyl alcohol units could present certain solubility in water, and in case of being administered orally in a systematic way they could be partially absorbed. It is well-known that polyvinylalcohol can originate renal dysfunction. The copolymers with more than 30 % of vinyl alcohol units, are not good soluble in organic solvents which makes more difficult the drying step at the pharmaceutical industry, because of the need of using water (or mixtures of organic solvents and water) to dissolve these copolymers.

The occlusion of an active substance, drug or substance in general within a matrix of some of the polymers claimed in this patent, can be performed by any of the following ways:
a) depositing the polymer on the substance, solid or liquid (adsorbed or absorbed in the latter case on or in an inert filler), wetting the other components of the formulation with a polymer solution and evaporating thereafter the solvent.
b) intimate mixing of the polymer as powder or as granulate, with a substance, active substance or drug, solid or liquid, supported in the latter case on an inert solid.
c) Intimate mixing of the solids, humectation with solvent (preferably acetone or ethanol ), and subsequent drying.

The so obtained products can be granulated or compressed with addition or not of other excipients as fillers, lubricants etc.

For equal residual water content, equal composition of the other components of the solid formulation and equal content of polymer or copolymer the retardatory power of the release rate of active substances grows in the following manner:
polyvinylacetate< copolymers with blockcharacter and low content of vinyl alcohol units <copolymers with block character and higher content of vinyl alcohol units. This behavior is inverse to the behavior of other copolymers constituted of a very hydrophilic comonomer and a non so hydrophilic comonomer. For these copolymers the higher is the content of the very hydrophilic comonomer the higher is the swelling rate and the release rate of occluded substances

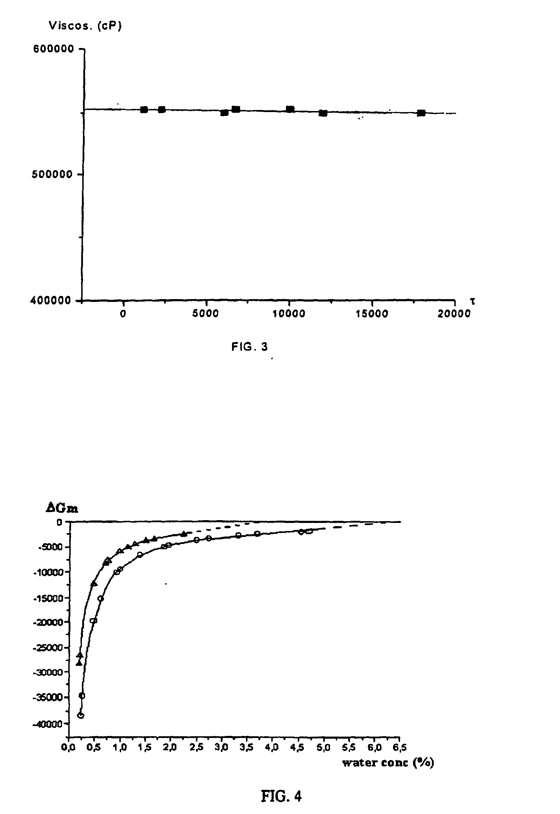

In the case of the polymers which pharmaceutical use is here proposed, the studies made show that:
a) The diffusion coefficients of water in the polymer are much smaller for the copolymers than for the polyvinylacetate, decreasing as the vinyl alcohol monomeric unit content is increased, within the range of composition of the copolymers which use is claimed in this patent. Cause of this behavior is the proven formation of water clusters during the diffusion of water into the polymers (Q.T. Nguyen y cols; Journal of Membrane Science, 113 (1) 137-150, (1996)).
b) The values of the free energy of mixing (ΔGₘ₎ of water-polymer mixed phases allow to compare the compatibility of the different polymers toward water. At ΔGₘ = O the water polymer mixed phase becomes unstable and phase separation occurs. In Fig. 4 are shown the values of ΔGₘ for polyvinylacetate (Mw 537000 ± 5750) and a copolymer with 12 molar % of VA units in dependence of the water content of the polymer. As can be seen in Fig. 4 the extrapolation to ΔGₘ = 0 shows that phase separation occurs in both cases at relatively low water concentrations. The copolymer presents, as expected, a higher water compatibility and therefore the water-polymer mixed phase suffers phase separation at higher water contents.

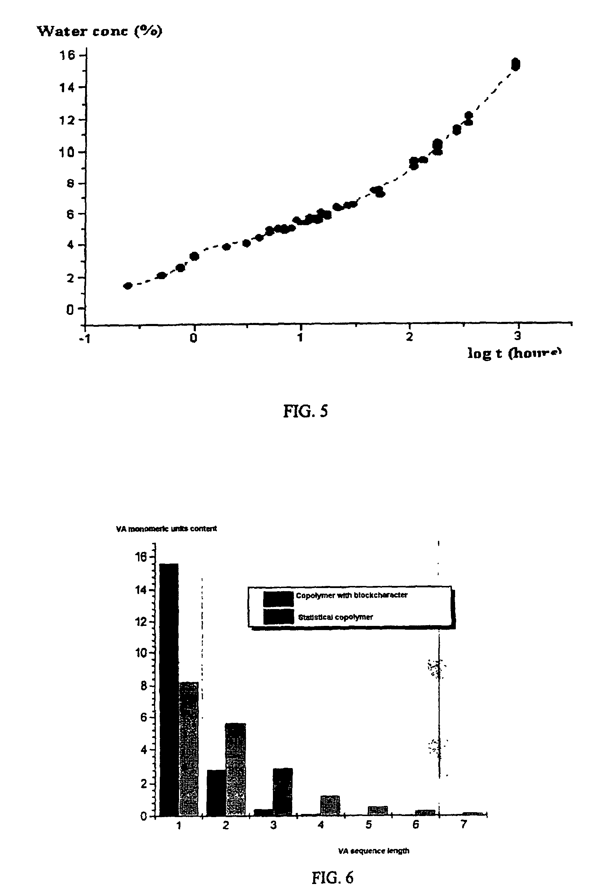

These results are corroborated by an experiment of swelling of a rectangular film (0.5 g weight and 0.5 ± 0.0059 mm width) of PVAc (Mw 537000 ± 5750) perfectly dry. The film dipped in water was periodically weighed after drying the surface over a period of 40 days. Fig. 5 shows the results of this experiment. It can be seen that after approximately one hour there is a change in the water swelling kinetics, at a water concentration in the polymer of about 3.5 %, a value very close to the extrapolated concentration value for ΔGₘ = 0 in Fig. 4 the same polymer (3.7 %).

Optical microscopy of a thin film of PVAc on a glass slide dipped in water and kept under water corroborated a phase separation process, which started after prolonged immersion and led to the formation of droplets, probably constituted of almost pure water. Since the polymer- rich phase has a negative ΔGₘ it takes water again, from the surrounding water and from the water droplets as well. The process of phase separation is repeated then again and again, and resulting of this the water droplets become smaller but their number increases in such a way that , within a relatively short period of time , they can contact and build water channels.

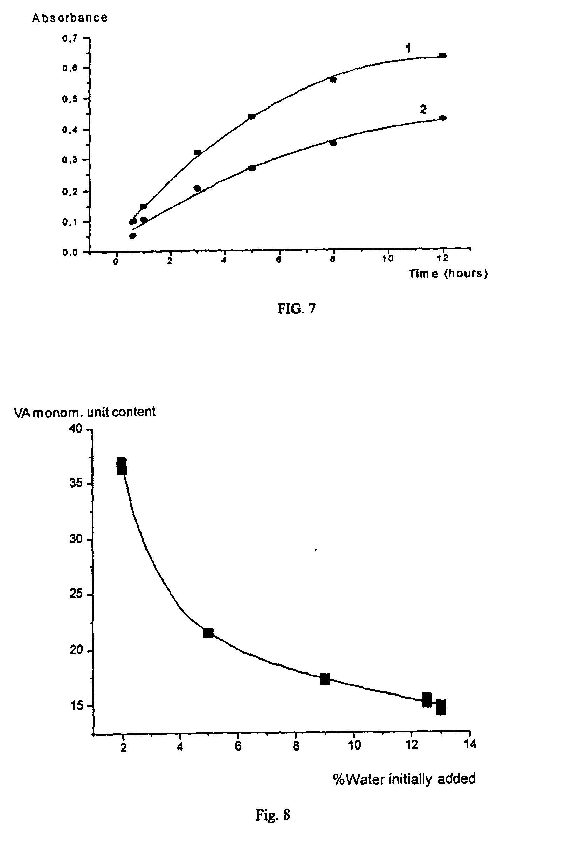

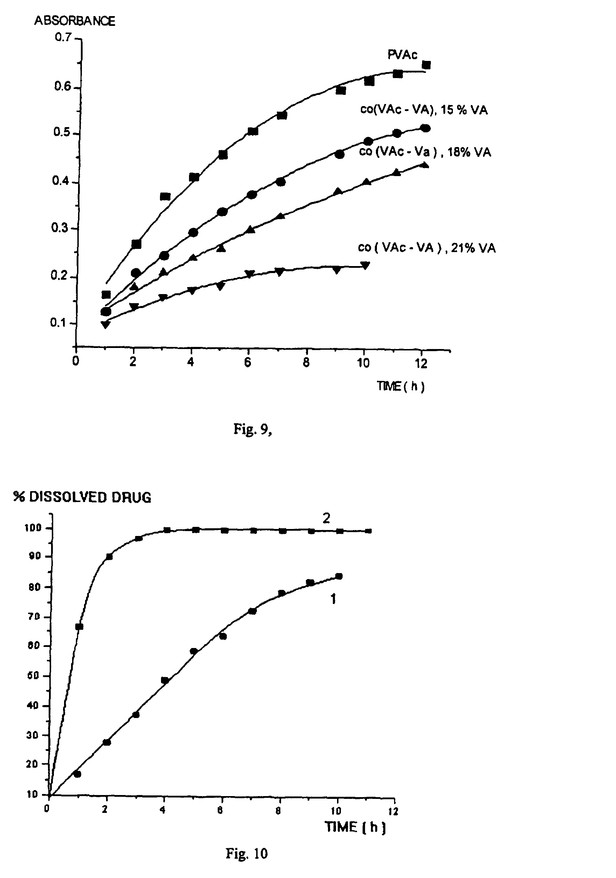

The copolymer presents, as expected, a higher water compatibility and therefore the water-polymer mixed phase suffers phase separation at higher water contents, and since the diffusion coefficients for the copolymer are smaller, this process occurs after longer time than it is the case for PVAc.
c) The block character of several copolymer samples (21, 36 and 43 molar % of VA units) obtained by different alkaline alcoholysis and acid hydrolysis conditions was determined. ¹³NMR spectra of the samples in D₃CCOCD₃/H₂O/HMDS were obtained with a 400 MHz spectrometer. The three dyad signals of the methylenic carbons at 45- 38 ppm were used to calculate this parameter (D.C.Bugada, A. Rudin; Polymer, Vol 25, 1759 (1984). The calculated persistence ratio showed values between 1 (statistical copolymer) and 2.1 (copolymer with block character. The sequence length distribution was determined using the dyad signals of the methylenic carbons and the expression developed by Flory originally relating the sequence length distribution with the melting temperature of crystalline copolymers.(Flory P. J. , Trans. Faraday Soc., 51, 848 , (1955)). In Fig. 6 are shown the sequence length distributions of two copolymers with 21 molar % content of VA units, one statistical (obtained by acid hydrolysis) and the other with block character. With these two copolymers were made tablets of theophylline with the same composition, shape, weight and hardness. Fig. 7 shows the in vitro theopylline release curves of the two types of tablets. An Erweka Dissolution Tester) built in accordance to USP regulations, with 6 test stations (paddles, 100 rpm, 900 ml water in each vessel, 37 °C), was used for theophylline release experiments. Samples were withdrawn every hour and their absorbance at 270 nm was measured after proper and equal dilution. For each type of tablet were performed at least three release tests. The release curves shown in Fig. 10 evidence a considerable influence from the monomeric sequence distribution
d) Using the intercept method (W. Mannchen; "Einfuehrung in die Thermodynamik der Mischphasen" VEB Deutscher Verlag fuer Grundstoffindustrie, Leipzig, DDR, (1965)), were determined the partial molar volumes of the monomeric units of the copolymers of Fig. 7 at 28, 30, 34 and 37 °C.. It was stated that the partial molar volumes were perfectly additive for both copolymers. These results indicate that there is no difference between the partial molar volumes of the monomeric units of two copolymers of equal composition and different sequence distribution. Since the volume of a substance is composed by the sum of the occupied volume, the interstitial free volume and the hole free volume, the only possibility that the hole free volumes in the two mentioned copolymers differ is that the sum of the interstitial free volume and the hole free volume for both copolymers in all studied temperatures is exactly the same value. The probability of this does not seem to be high. This allows almost to discard that the differences in the release rate of the occluded substance is owed to differences in the hole free volume of the two copolymers.
e) Differential scanning calorimetry analysis of copolymer samples with less than 25 molar % vinyl alcohol monomeric units content and very different thermal history showed that the formation of crystalline microdomains is not a factor of considerable importance that could explain the differences found in the water diffusion rates in polyvinylacetate and in the copolymers. The thermogram of a copolymer sample with 21 molar % of VA units (P=1.63) treated at 80 ° C under vacuum during 64 hours did not showed a melting peak. The same happened with other thermal treatments. Only a sample initially gelled in methylacetate and dried under vacuum at 60 ° C during 6 hours showed a melting peak (1,67 J/g). Using the value ΔHu = 7.1 kJ/g, (B. Wunderlich; Macromolecular Physics, Academic Press N.Y. <A> Vol. 3 table VIII, 6 (1980)) could be calculated the % of VA units forming the crystalline microdomains (8,64 %). This means that even in this case only 0.9 % of the total mass of the copolymer was constituted by the crystalline microdomains. For copolymers with less than 21 molar % of VA units was impossible to detect any melting peaks in samples with very different thermal history. On the contrary in copolymers with more than 25 molar % of VA units is common the presence of crystalline microdomains.

Considering the discussed information it can be concluded that the differences in the values of the diffusion coefficients of water for the homo and copolymer are mainly due to the different tendency for water cluster formation of these substances and not to the existence of crystalline microdomains in the studied copolymer samples. The main controlling factors for the delivery of occluded substances in the studied polymers are the mentioned tendency of formation of water clusters in the initially formed water-polymer mixed phase and the water-polymer compatibility. Latter factor determines that phase separation occurs at a definite water content of the initially formed mixed phase. The phase separation process concludes with the formation of water channels through which the diffusion of occluded substances is facilitated. Differences in sequence length distribution in the studied copolymers affect the delivery rates of occluded substances. This is probably due to the previous discussed factors, but not to difference in hole free volumes.

Another important conclusion derived from the knowledge of the controlling mechanism of the delivery of occluded substances is that moisture or any residual organic solvent initially present in the polymer can affect the release rate of an occluded substance.

### • First example

### Obtaining of polyvinylacetate, adequately pure and innocuous, at industrial scale.

According to the description of the process exposed in this patent, using a reactor of 250 liters with double control of temperature and very efficient cooling, ethanol 95 % as solvent (100 lt), 537.5 g dibenzoylperoxide, performing the reaction at 80 °C for 8 hours, and purifying and drying the product as described, 83.5 Kg of polyvinylacetate (89.9% yield) were produced, having the following specifications:
- average molecular mass: Mw = 25145 ±1020 daltons Mn = 9365 ± 410 daltons
- glass transition temperature: 37,6 °C
- humidity: 0.651% en peso
- remnant monomer : 0.2 ppm
- total acidity referred as acetic acid: 0,24 %
- ashes: 0,0085 % en peso
- dibenzoylperoxide : 0,0 %

### • Second example

### Use of polyvinylacetate as binder in a propanolol formulation.

A granulate was first prepared using polyvinylpirrolidone (PVP) as binder (molecular mass 25500 daltons). The polymer was mixed with the rest of the excipients and ethanol was used as moistening agent insuch a quantity and the necessary time so that the powder was adequately humected and agglomerated. The drying was performed in a fluidized bed until an absolute residual moisture of 1.8 % was reached. The obtained granulate constitutes the Formulation 1 and is used for comparison purposes.

In the same previous described manner was prepared the granulate of the Formulation 2 using polyvinylacetate (Mw= :26436 □ 545 daltons) instead of PVP. The PVAc used was a powder stabilized with lactose in a 50:50 ratio.

Formulation 3 was obtained from a granulate prepared using also PVAC as binder. The PVAc was dissolved in ethanol, having the solution a concentration of 38 % by weight. The solution was mixed with the rest of the excipients and with an additional quantity of ethanol, processing the mixture as described for Formulation 1.

In Table 1 are shown the compositions of the three formulations

**Table 1.**

| Tested propanolol formulations. | | | |
|---|---|---|---|
| Components (mg) | Form.1 (binder:PVP) | Form.2 (binder: solid PVAc with lactose) | Form.3 (PVAc in solution.) |
| Propranolol HCL | 40.00 | 40.00 | 40.00 |
| Lactose | 35.00 | 35 .00 | 35.00 |
| Maize starch | 15.00 | 15.00 | 15.00 |
| PVP | 3.00 | ----- | ----- |
| PVAc | ----- | 3.00 | 2,30 |
| Explotab | 3.00 | 3.00 | 6.00 |
| Talc | 3.00 | 3.00 | 6.00 |
| Magnesium stearate | 2.00 | 2.00 | 2.00 |
| Ethanol (µL) | 42.00 | 43.00 | 43.00 |

The characteristics of the granulates obtained with the different tested formulations are reported in Table 2.

**Table 2.**

| Physical and technological characteristics of the granulates | | | |
|---|---|---|---|
| Parameters | Form. 1 (binder : PVP) | Form.2 (binder: solid PVAc with lactose) | Form.3 (PVAc in solution) |
| Granular density g/cm³ | 0.43 | 0.43 | 0.44 |
| Granular density after normalized vibration g/cm³ | 0.48 | 0.48 | 0.51 |
| Repose angle (degrees) | 29.00 | 29.50 | 29.60 |
| Flow rate (g/cm²seg) | 10.30 | 10.80 | 11.30 |
| Carr index % | 10.40 | 11.30 | 13.70 |
| Haussner index | 1.11 | 1.12 | 1.15 |
| Average diameter µm | 540.00 | 570.00 | 290.00 |
| Flow quality | continuous | continuous | continuous |
| Grain friability % | 11.60 | 13.50 | 23.70 |

As can be concluded from the values in Table 2 all three formulations showed good properties in general. The values of the Carr Index are below 15 %, what is considered proper. The Haussner Indexes are below 1.25. The values of the flow rate are convenient: The repose angles are smaller than 30 degrees. This properties guarantee good flow of the granulates through the hopper when they are compressed avoiding the scattering of the weights of the tablets. The granulates show good mechanical strengh with granular friability smaller than 40 %.

The granulates were compressed using a die of 6.35 mm diameter and flat faced bevelet edge punch. In Table 3 are shown the properties of the tablets.

**Table 3-**

| Physico-mechanical and technological properties of the propanolol tablets | | | | |
|---|---|---|---|---|
| Parameters | Limits | Formulation 1 | Formulation 2 | Formulation 3 |
| Mass (mg) | 100.00 ± 5.50 | 103.75 ± 2.07 | 103.65 ± 1.58 | 99.31 ± 1.39 |
| Height (mm) | 2.50 ± 0.1 | 2.65 ± 0.04 | 2.65 ± 0.02 | 2.54 ± 0.02 |
| Hardness (Monsanto)(Kg) | 4.50 ± 0.5 | 4.65 ± 0.31 | 4.50 ± 0.37 | 4.55 ± 0.47 |
| Friability % | < 1 | 0.06 | 0.09 | 0.13 |
| HFR | > 1.5 | 77.50 | 11.25 | 30.76 |
| Disintegration min. | < 15 | 4 | 7 | 4 |

The stability of the tablets was tested after 12 months. The measured values are reported in Table 4 and Table 5.

**Table 4-.**

| Physico- mechanical and technological properties of the propanolol tablets after 12 months | | | | |
|---|---|---|---|---|
| Parameters | Limits | Formulation 1 | Formulation 2 | Formulation 3 |
| Mass mg | 100.00 ± 5.5 | 103.50 ± 1.62 | 103.25 ± 0.77 | 99.30 ± 1.22 |
| Height mm | 2.55 ± 0.1 | 2.64 ± 0.03 | 2.65 ± 0.01 | 2.55 ± 0.02 |
| Hardness (Montsanto)(kg) | 4.50 ± 0.5 | 4.95 ± 0.42 | 4.90 ± 0.52 | 4.93 ± 0.52 |
| Friability % | < 1 | 0.11 | 0.18 | 0.37 |
| HFR | > 1.5 | 47.72 | 29.88 | 12.16 |
| Disintegration min. | < 15 | 2 | 2 | 2 |
| Organoleptic properties | White shining tablets | Satisfies | Satisfies | Satisfies |

**Table 5.**

| Propanolol tablets after 12 months | | | | |
|---|---|---|---|---|
| Parameters | Limits | Formulation 1 | Formulation 2 | Formulation 3 |
| Description | White, flat, bevelet edge tablets. | Satisfies | Satisfies | Satisfies |
| Identification | Chromatographic. One single peak with same retention time as the retention time of the standard. | Satisfies | Satisfies | Satisfies |
| % of nominal dose | 90-110 | 100.14 | 100.48 | 98.54 |
| Dissolution % | Not less than 75 % of the nominal dose in 30 min. | | 99.50 | 99.57 |
| | | | 98.13 | 100.73 |
| | | | 97.54 | 99.73 |
| | | | 98.75 | 99.96 |
| | | | 100.40 | 99.58 |
| | | | 99.72 | 100.50 |
| Disintegration (min) | Less than 15 minutes in water at 37 ° C. | 2 | 2 | 2 |

The shown results indicate that the polyvinylacetate confers adequate properties to the tested granulates and tablets in which this polymer was used as binder.

### • Third example

### Copolymers of vinyl acetate- vinyl alcohol with predetermined composition

Several polyvinylacetate alcoholysis reactions were performed in which the reaction temperature was 40 ° C and the mass ratio of polyvinylacetate, sodium hydroxide and ethanol in the initial reaction mixture was the same. The said ratio was; PVAc: NaOH: ethanol = 25: 0.36: 53,2. The PVAc was dissolved in part of the ethanol, the sodium hydroxide in the other part of the ethanol. To both solutions was added such a quantity of water that when both solutions were mixed at the reaction temperature, the quantity of water present in the mixed solution represents a quantity in the range of 0 to 15 % of the total quantity of solvent. By all reactions, samples of the reaction mixture were withdrawn at different times. Those samples were neutralized with acetic acid. It was observed that the content of vinyl alcohol monomeric units in the copolymers in all cases did not change after a reaction time of 20 minutes and that the content of vinyl alcohol units in the copolymers depended on the quantity of water initially added. This is shown in Fig. 8.

### • Fourth example

### Theophylline tablets differentiated only by the composition of the polymers of the matrix.

Several granulates were prepared by wet granulation using solutions of different polymers. All granulates had the same composition; monohydrated theophylline (86 %), polymer (13 %), magnesium stearate (1 %), and were different only by the monomeric composition of the polymers. These polymers were; polyvinylacetate and copolymers with 15,18 and 21 molar % of vinyl alcohol monomeric units content.

The polyvinylacetate showed (by S.E.C.) an average molecular weight, Mw, of 25175 daltons and the copolymers were obtained by means of alkaline alcoholysis of the former homopolymer.

Using the granulates were prepared 4 types of tablets. Some of the most important parameters for the characterization of the granulates and tablets are shown in Table 6.

**TABLE 6.**

| Main physical parameters of the granulates and tablets. | | | | | |
|---|---|---|---|---|---|
| Polymer | Moisture in granulate (%) | Friability of tablets (%) | Hardness (Monsanto)( Kg) | Weight (mg) | Height (mm) |
| | | | | | |
| PVAc | 1,6 | 0,2 | >11 | 128,4 | 3,14 |
| Cop., 15%VA | 1,8 | 0,1 | >11 | 129,5 | 3,15 |
| Cop., 18%VA | 1,8 | 0,1 | >11 | 130,5 | 3,04 |
| Cop., 21%VA | 0,8 | 0,2 | >11 | 131,1 | 3,17 |

Dissolution experiments in vitro were performed with the different tablets, using a dissolution tester Erweka T6 with 6 vessels, using 900 ml 0.1 N HCl as dissolution medium in each vessel, paddles (100 rpm) and a temperature of 37 °C. Samples were withdrawn every hour and their absorbance at 270 nm was measured after proper and equal dilution. For each type of tablet were performed at least three release tests. The release profiles are shown in Fig. 9. It results evident the influence that exerts the monomeric composition of the polymers upon the release rate.

### • Fifth example

### Sustained release acetyl salicylic acid tablets

Tablets were made using polyvinylacetate, obtained as is described in the first example of this patent, as controlling matrix in powder form and granulation by means of moistening with solvent.

Composition of the formulation:
- Acetyl salicylic acid 82 %
- Polyvinylacetate 15 %
- Maize starch 3 %

Three lots of tablets (42.6 Kg each) were made with the described composition using the following procedure:
1- Milling of PVAc in a hammer mill, slow speed, sieve 183.
2-Mixing of 36 Kg. of acetyl salicylic acid and 6.6 Kg of milled gross grain PVAC.
3-Milling of the former mixture in a cutter mill, medium speed, sieve A1.
4-Moistening of the milled mass with acetone
5-Granulation in a cutter mill, slow speed, sieve 185.
6-Drying in fluidized bed at 50 ° C for 25 minutes. Residual moisture 0.2 %.
7-Addition of maize starch and mixing.
8- Degranulation in a cutter mill, medium speed, sieve 183. Process loss 2.8 %.
9- Compression for tablets, weight of each tablet 732 □ 18 mg, Hardness (Monsanto) 0.5 Kg.

The in vitro release of the tablets was effected in a dissolution tester Erweka DT-6 ( basket, 100 rpm), at 37 ° C using phosphate buffer (pH=6.8) as dissolution medium ( 900 ml). The concentration of acetyl salicylic acid in the dissolution medium was determined, taking samples at different time intervals. The samples were alkalinized, conveniently diluted and measured spectrophotometrically at 308 nm. After 12 hours the drug was completely released at 90° C.

In the same manner were tested commercial tablets (Bayer 8 hours) for comparison. Three tablets were released for each formulation. The average release profiles are shown in Fig. 10.

The results of a pharmacokinetic study with 13 non smokers adult volunteers are shown in Fig

11. The administered dose was 600 mg. The plasma concentration of acetyl salicylic acid was measured using HPLC. The plasma samples were previously deproteinized using acetonitrile as deproteinizing agent.

### • Sixth example

### Sustained release nifedipine tablet.

A formulation was prepared with the following composition:

| | |
|---|---|
| Nifedipine | : 20 mg |
| Co (VAc-VA) 20 % Hydr. | 4.2 mg |
| Monohydrated lactose | 4.2 mg |
| Microcrystalline cellulose | 86.4 mg |
| Maize starch | 4.0 mg |
| Magnesium stearate | 1.2 mg |

The copolymer used was a powder mixed with lactose in a 50:50 ratio. All products were initially mixed with exception of the nifedipine. This product was dissolved in warm acetone and the solution was used to moisten the solid mixture. The mixture was granulated and the granulate showed adequate parameters and continuous flow during the compression. The tablets presented a hardness (Monsanto) of 6.8 □ 0,3 kgf, a friability of 0,16 % □ 0,01 % good organoleptic properties and low weight scattering.

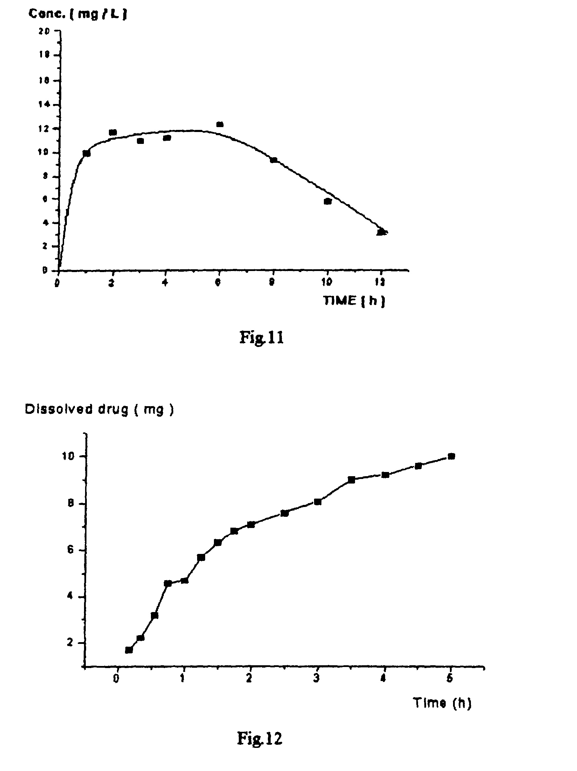

The in vitro release testing was performed using the apparatus 4 described in the USP XXIII, flow rate 4 ml /minute, HCl pH=1.2 as dissolution medium for the first two hours and later buffer pH= 6.8. The sample quantification was made spectrophotometrically at 238 nm. The results are shown in Fig. 12. The release curve is very similar to the release curve in same conditions of a renown commercial product.

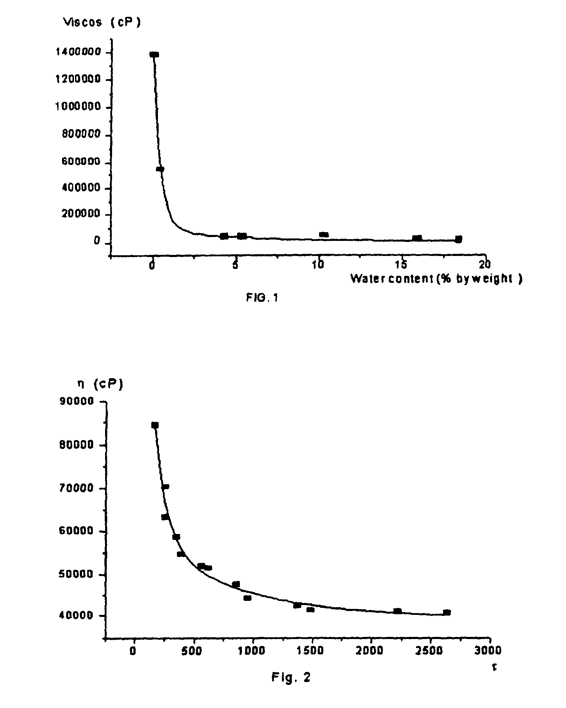

Fig. 1:Dependence of the viscosity with the water content in the PVAc at 100 °C, average molecular mass of the polymer MW= 27500 daltons.

Fig. 2: Dependence of the viscosity with the shear stress for PVAc with 5 weight % water content. Temperature 100° C, average molecular mass Mw=27500 daltons

Fig. 3: Dependence of the viscosity with the shear stress for PVAc with0.46 weight % water content. Temperature 100 ° C, average molecular mass Mw= 20500 daltons.

Fig. 4: Free energy of mixing of the PVAc-water mixed phase (Δ), and co (VAc-VA)- water mixed phase (o), in dependence of their compositions.

FIG. 5: Swelling of a PVAc film in water.

Fig.6: Sequence length distribution of two copolymers with 21 molar % of VA monomeric units. One statistical and the other with block character (P= 1.63).

Fig. 7: Theophylline release, 124 mg tablets, copolymer 13%, Mg.stearate 1 %.

Curve 1: statistical copolymer with 21 molar % of vinyl alcohol units.

Curve 2: copolymer with block character and 21 molar % of vinyl alcohol units.

Fig 8: Dependence of the monomeric composition of the copolymers with the initial water content of the reaction mixture.

Fig. 9: Theophylline release
Curve 1: tablet with PVAc.
Curve 2: tablet with copolymer (15 % molar of VA units).
Curve 3: tablet with copolymer (18 % molar of VA units).
Curve 4 :tablet with copolymer (21 % molar of VA units)

Fig. 10: Release profiles of acetylsalycilic acid
Curve 1: Tablet with 15 % PVAc
Curve 2: Bayer (8 hours)

Fig. 11: In vivo release of acetylsalicylic acid tablets. PVAc 15 % of the formulation.

Fig. 12: Dissolution profile of nifedipine in apparatus 4.

## Claims

1. Procedure for the obtaining of polyvinylacetate with average molecular mass between 10000 and 40000 daltons, remnant monomer content less than 2 ppm by weight, water content less than 1.5% by weight, innocuous after oral ingestion, obtained by polymerization of vinyl acetate in solution, preferably in ethanol, or in suspension, with use of dibenzoyl peroxide as initiator and that is characterized by:
- the purification of the polymer by means of the addition of an adequate volume of hot water to a solution of the polymer (in ethanol or other volatile solvent), or by means of the addition of a solution of the polymer to an adequate volume of hot water (> 80 ° C), during a time that depends on the employed equipment, on the slow mechanical stirring, and on the maintenance of a high temperatura (> 80 ° C) of the resultant mass, with optionally bubbling of purified air through the said mass , until the achievement of the separation of the solvent by means of evaporation or dragging with help of the water vapor, the decomposition of the remnant initiator, the dissolution of the major part of the benzoic acid in water and the removal of the major part of the remnant monomer, finally the water phase is separated from the semisolid humid polymer mass.
- the drying of the semisolid humid polymer mass by means of heating of the said mass at a temperatura between 80 and 140 °C, under vacuum (0.02-13 kPa), and stirring the mass slowly until the polymer contains a humidity less than 1.5 % by weight and remnant monomer less than 2 ppm.

2. Procedure for the obtaining of vinyl acetate- vinyl alcohol copolymers with less than 30 molar % of vinyl alcohol monomeric units by means of alkaline alcoholysis/ hydrolysis, characterized -by:
- the use an alcohol, preferably ethanol, as solvent, and the fixing of a ratio between the initial quantities (by weight) of polyvinylacetatel alcohol/ alkaline hydroxide within the range of ratios that following the same order is expressed in the ratio: 50-80 / 130-170 / 1.
- the addition of water to the initial reaction mixture in such quantities that it constitutes between 4 and 25 % of the solvent present.

3. Procedure for the obtaining of polyvinylacetate and of vinyl acetate- vinyl alcohol copolymers as a powder, stable enough in its storage and useful in preparations of compressed tablets obtained by direct compression or by means of previous wet granulation, characterized by being performed in three steps, the first of which is the milling of the polymer in a cutting or hammer mill, using a sieve with hole dimensions between 2.5 and 0.8 mm, the second step is the mixing of the gross grained polymer, obtained in the described first step, with pharmaceutical crystalline excipients of small particle size (preferably lactose) or with a crystalline active substance, followed by the milling of the mixed solid in the same kind of mill using a sieve with hole dimensions between 0.2 and 0.04 mm, the third step is the homogenizing mixing of the milled product.

4. Polyvinylacetate with average molecular mass Mw between 10000 and 40000 daltons as the only or principal mean of agglutination in procedures for the obtainment of base granulates, suitable to be used in direct or double compression of tablets, or of granulates that contain active substances, being the said granulates characterized by:
- being obtained using polyvinylacetate powder intimately mixed with lactose or with other crystalline excipient by means of moistening with acetone, ethanol or other appropriate solvent.
- being obtained from solid excipients and a polyvinylacetate solution in an organic convenient solvent.
- to contain polyvinylacetate between 1 and 10 % by weight.

5. Polyvinylacetate of average molecular mass, Mw, between 10000 and 40000 daltons and vinyl acetate- vinyl alcohol copolymers with less than 30 molar % of vinyl alcohol monomeric units, as binders and controlling substances of the release of active substances in compressed tablets and pellets by means of the formation in the mentioned solid forms of controlling polymeric matrixes, characterized by:
- being constituted mainly by the mentioned polymers (>60 weight % of the polymeric matrix and between 2 and 25 weight % of the formulation),
- the fact that the occlusion of the active substance, the drug or the 10 releasing substance in general, within the matrixes takes place by either one of the followig ways :
a) depositing the polymer on the substance, solid or liquid (adsorbed or absorbed in the latter case on or in an inert filler), wetting the other components of the formulation with a polymer solution and evaporating thereafter the solvent.
b) intimate mixing of the polymer as powder or as granulate, with a substance, active substance or drug, solid or liquid, supported in the latter case on an inert solid.
c) Intimate mixing of the solids, humectation with solvent (preferably acetone or ethanol), and subsequent drying.
d) optional compression of the products obtained as formerly describes, with addition or not of other excipients as fillers or lubricants.
